# EUROPÄISCHE PATENTSCHRIFT

(11) **EP 1 778 084 B1**
(45) Veröffentlichungstag und Bekanntmachung des Hinweises auf die Patenterteilung: **05.03.2008**
(21) Anmeldenummer: 05781096.2
(22) Anmeldetag: 10.08.2005
(51) Int. Cl.: A61B 5/107, A61B 1/04

(54) **ENDOSKOPISCHES VIDEO-MESSSYSTEM**
ENDOSCOPIC VIDEO MEASURING SYSTEM
SYSTEME DE MESURE VIDEO ENDOSCOPIQUE

(30) Priorität: 19.08.2004 DE 202004012992 U
(43) Veröffentlichungstag der Anmeldung: 02.05.2007
(73) Patentinhaber: Storz Endoskop Produktions GmbH, 78532 Tuttlingen (DE)
(72) Erfinder: KRATTIGER, Beat, CH-8222 Beringen (CH); KLUMPP, Martin, 78532 Tuttlingen (DE); KUSTER, Manfred, CH-9443 Widnau (CH)
(74) Vertreter: Stamer, Harald
(86) Internationale Anmeldenummer: PCT/EP2005/008663
(87) Internationale Veröffentlichungsnummer: WO 2006/018199

(56) Entgegenhaltungen:
- US-A- 3 595 220
- US-A- 4 660 982
- US-A- 4 706 653
- US-A- 5 669 871
- US-A- 6 135 947

## Beschreibung

Die Erfindung betrifft ein endoskopisches Video-Meßsystem mit einem proximalen Bedienteil, einem Einführungsteil und einem daran ansetzbaren Wechselkopf, wobei das Bedienteil einen Anschluß zur elektrischen und optischen Versorgung des Systems enthält, im Einführungsteil optische Übertragungsmittel für die Objektbeleuchtung und elektrische Versorgungs- und Übertragungsleitungen für einen im distalen Endteil angeordneten elektronischen Bildsensor vorgesehen sind und der Wechselkopf optische Übertragungsmittel für die Objektbeleuchtung und die Objektabbildung enthält

Die Endoskopie ist ein Hilfsmittel zur Inspektion verborgener Hohlräume. Ihre Einsatzgebiete sind neben der Medizin auch in der Technik, z.B. in der Schadenanalyse oder in der Qualitätskontrolle.

In der Technik erfährt die Endoskopie eine wachsende Beliebtheit, da die verfügbaren Arbeitsdurchmesser laufend kleiner werden. Durch verbesserte Lichtquellen, Optiken und Materialien nimmt dabei die Bildqualität stetig zu. Das Aufkommen von miniaturisierten Komponenten in der Videotechnik verhalf der Endoskopie zudem zu weiterer Publizität.

Mit der wachsenden Verbreitung der Video-Endoskopie steigt auch das Bedürfnis, in verborgenen Hohlräumen geometrisch zu messen und zu manipulieren. Als wichtige Beispiele können die Länge von Ermüdungsrissen in Triebwerksschaufeln oder das Ausmaß von Korrosionsstellen in Wärmetauscherrohren von Kernkraftwerken angegeben werden.

Neben der Meßfunktion werden aber auch immer mehr modular aufgebaute, ausbaufähige Endoskopiesysteme gefordert. Diese Modularität ermöglicht günstige Grundsysteme, die bei Bedarf zu Meßsystemen aufgerüstet werden können. Diese Aufrüstung kann auch anwendungsspezifisch mit entsprechenden Spezialzubehören erfolgen. In der Praxis geschieht eine solche Aufrüstung zu Meßsystemen durch entsprechende Wechselköpfe und die zugehörige Computer-Software. Durch den modularen Aufbau steigt ferner auch die SystemVerfügbarkeit an, da defekte Teile (Wechselköpfe) ausgetauscht werden können.

Aus US 6 184 923 B1 ist ein Endoskop der eingangs genannten Art mit austauschbaren Köpfen bekannt. In einer Ausführungsform enthält der Kopf neben zwei Beleuchtungsoptiken zwei getrennte Objektive, welche auf einen gemeinsamen Bildsensor im Einführungsteil abbilden. Mit Hilfe der stereoskopischen Abbildung können auch Messungen am beobachteten Objekt ausgeführt werden.

Die Beleuchtungs- und Abbildungsrichtung kann in dem Kopf in direkter Verlängerung des Einführungsteils geleitet werden oder durch geeignete Umlenkmittel senkrecht zur Längsachse des Einführungsteils gewählt werden.

Aus DE 35 16 164 C2 ist ein Endoskop mit Okularbeobachtung bekannt, das mit auswechselbaren Köpfen als Adapter zur Längenmessung ausgestattet werden kann. In einer Ausführungsform sind in dem Kopf eine Beobachtungsoptik und zwei Projektionsoptiken mit zueinander parallelen optischen Achsen vorhanden. Über die Beobachtungsoptik wird im Okularteil ein linearer Bezugsindex dargestellt. Über die Projektionsoptiken werden Indexpunkte als Schatten auf die Objektebene projiziert. Die optischen Achsen der Projektionsoptiken sind so ausgerichtet, daß die Verbindungslinie der projizierten Indexpunkte parallel zum Bezugsindex im Okularteil ausgerichtet ist. Mit den bekannten Abständen der Indexpunkte zueinander und dem Abstand ihrer Verbindungslinie zum Bezugsindex können Längenmessungen im Sichtfeld durchgeführt werden. Aus der Lage der Indexpunkte zueinander im Sichtfeld kann der Abstand der Beobachtungsebene zum vorderen Ende des Adapters ermittelt werden. Wegen der als Schatten projizierten Indexpunkte ist ihre Erkennbarkeit im beleuchteten Sichtfeld wenig kontrastreich.

Aus DE 103 08 383.9 ist ein Video-Meßendoskop bekannt, bei dem die Funktionseinheiten Bedienteil, Schaft und Kopfteil unterschieden werden können, wobei diese Funktionseinheiten jedoch nicht austauschbar sind. Das Kopfteil enthält neben einem Beleuchtungsfaserbündel ein Video-Empfangssystem und ein Projektionssystem zur Erzeugung eines Meßmusters auf einem Objektfeld. Das Projektionssystem wird z.B. über eine Singlemode Glasfaser mit Laserlicht versorgt. Die Glasfaser ist vom Bedienteil bis in den Kopfteil hinein geführt. Die Lichtaustrittsfläche liegt im Brennpunkt einer Kollimationsoptik zur Erzeugung eines kollimierten Meßstrahlenbündels. Über eine diffraktive Optik wird aus dem Meßstrahlenbündel eine Vielzahl nach mehreren Richtungen ausfallender, kollimierter Teilstrahlenbündel gebildet, die über eine Projektionsoptik als leuchtende Punkte auf das Objektfeld abgebildet werden.

Der Vorteil des Einsatzes einer diffraktiven Optik besteht darin, daß sich damit auch feinststrukturierte Projektionsmuster zur Erhöhung der Meßgenauigkeit erzeugen lassen. Die Verwendung von Laserlicht ergibt eine sehr gute Erkennbarkeit der Projektionsmuster. Aufgrund der Kollimation der Meßstrahlenbündel können die Projektionsmuster über einen großen Abstandsbereich zum Objektfeld dargestellt werden.

Eine erste Trennmöglichkeit von Schaft und Kopfteil könnte darin bestehen, daß die Singlemodefaser und die Beleuchtungsfasern an der Trennstelle eingegossen und anschließend für verlustarmen Lichtübertritt abpoliert werden. Diese Trennmethode benötigt zwar ein Minimum an Schaftquerschnitt, sie bedingt aber, daß die Kernbereiche der Singlemodefasern beim Anfügen mit einer Präzision von ca. 0,3 µm aufeinander treffen müssen. Ist die Abweichung größer, tritt wenig oder kein Licht mehr durch die Singlemodefaser. Eine solche Präzision erfordert einen übermäßigen, kaum realisierbaren konstruktiven Aufwand, vor allem wenn man beliebige Schäfte mit beliebigen abgetrennten Kopfteilen kombinieren will.

Eine zweite Tennmöglichkeit besteht wiederum in einem vergossenen und abpolierten Schaftende. Das Beleuchtungslicht wird z.B. durch Fasern oder Glaskörper im abgetrennten Kopfteil übernommen. Das Kopfteil beinhaltet ferner zum Empfang des Meßlichtes eine Kollimationslinse. Beim Ankoppeln des Kopfteiles kommt das Ende der eingegossenen Singlemodefaser am Schaftende in den Brennpunkt der Kollimationslinse zu liegen, deren ausgehendes Strahlenbündel die Richtung des Projektionsmusters definiert.

Aus den vorstehenden Überlegungen wird deutlich, daß die Montage der beiden aufgetrennten Teile zueinander ebenfalls verschiebungsspielfrei erfolgen müßte, was einen großen Konstruktionsaufwand erfordert. Verschiebungen des Kopfteiles durch Kräfte, Stöße oder beim Wechsel hätte hier Winkelverschiebungen des Projektionsmusters zur Folge, wodurch Meßfehler entstehen. Bei der Anfertigung von einzelnen Kopfteilen müßte die durch den Schaft vorgegebenen gegenseitige Lage der Faser- Aus- und Eintrittsflächen ebenfalls sehr genau eingehalten werden. Wegen dieser zu erwartenden technischen Schwierigkeiten wurde eine Auswechselbarkeit des Kopfes an dem bekannten System bisher nicht realisiert.

Der Erfindung lag die Aufgabe zugrunde, für ein Video-Meßendoskop die Möglichkeit des Ansetzens austauschbarer Kopfteile zu schaffen, mit denen sowohl einzelne Meßpunkte als auch mehrere Meßpunkte in Parallelprojektion sowie Vielpunktemuster in einfacher Weise und mit hoher Erkennbarkeit darstellbar sind und wobei die Anforderungen an die Positioniergenauigkeit des Kopfteiles gegenüber dem Schaftteil hohe Toleranzen erlauben.

Diese Aufgabe wird bei einem endoskopischen Video-Meßsystem der eingangs genannten Art dadurch gelöst, daß im Einführungsteil zur Übertragung einer Meßstrahlung eine Singlemode-Glasfaser vorgesehen ist, der ein im distalen Endteil des Einführungsteiles angeordnetes optisches System zur Erzeugung eines kollimierten Meßstrahlenbündels zugeordnet ist. Vorteilhafte Weiterbildungen ergeben sich aus den Merkmalen der Unteransprüche.

Das als Folge der erfindungsgemäßen Verlagerung der Kollimation des Meßstrahlenbündels in das Einführungsteil nunmehr in den Wechselkopf übertragene kollimierte Meßstrahlenbündel kann auch ohne weitere optische Abbildungsmittel auf das Objektfeld gerichtet werden. Weil der Meßpunktedurchmesser über den gesamten Arbeitsbereich möglichst klein sein soll, kann das Meßstrahlenbündel auch mit einer Kollimationsoptik von geringem Durchmesser erzeugt werden. Der zur Erzeugung des kollimierten Meßstrahlenbündels erforderliche zusätzliche Querschnittsbedarf am distalseitigen Ende des Einführungsteils kann auf diese Weise gering gehalten werden.

Bei der Montage der Wechselköpfe ist es wichtig, daß die Richtungen der Projektionsstrahlenbündel unabhängig vom Spiel des Verschlußmechanismus und von Herstelltoleranzen sind. Dabei können Verkippungsfehler durch die relativ großen planen Auflageflächen ausgeschlossen werden. Seitliche Verschiebungsfehler können jedoch nicht ausgeschlossen werden. Sie haben innerhalb des kollimierten Meßstrahlenbündels aber keinen Einfluß auf die Richtung und den Querschnitt des Meßstrahlenbündels, da der freie Durchmesser der Optik im Wechselkopf genügend viel größer als der Durchmesser des Meßstrahlenbündels ist. Auch longitudinale Verschiebungen aufgrund nachfolgender optischer Umlenk- und Teilermittel im Wechselkopf sind in dem System unkritisch für die Beibehaltung der Richtung der optischen Achse, was für die Anordnung von optischen Beugungs- und Projektionsmitteln im Wechselkopf mehr Freiheitsgrade ermöglicht.

Durch das erfindungsgemäße Konzept des kollimierten, parallelen Lichtübertritts durch die Schnittstelle zwischen distalem Endteil des Einführungsteiles und Wechselkopf muß ein angesetzter Wechselkopf nicht auf den 1/100 Millimeter genau seitlich positioniert werden. Wichtig ist lediglich, daß die Rotation und die Verkippung zur optischen Achse übereinstimmen, d.h. daß der Wechselkopf eben aufliegen liegen muß und die Rotationslage z.B. durch gegenüber stehende Bajonett-Zapfen spielfrei definiert wird.

In der Zeichnung sind Ausführungsbeispiele des Meßsystems schematisch dargestellt und werden anhand der Figuren näher beschrieben. Dabei zeigen:
- Fig.1: die Sonde eines endoskopischen Video-Meßsystems in Ansicht,
- Fig.2: einen Querschnitt durch den Einführungsteil im mittleren Bereich,
- Fig.3: einen Querschnitt durch den distalen Endbereich des Einführungsteils,
- Fig.4a/b: eine Aufsicht und einen Längsschnitt am austrittseitigen Ende des Einführungsteils,
- Fig.5a/b: einen Querschnitt durch einen Wechselkopf in 0°-Durchführung und mit 90°-Umlenkung für ein Einstrahl-Meßsystem,
- Fig.6a/b: einen Querschnitt durch einen Wechselkopf in 0°-Durchführung und mit 90°-Umlenkung für ein Parallelstrahl-Meßsystem,
- Fig.7a/b: einen Querschnitt durch einen Wechselkopf in 0°-Durchführung und mit 90°-Umlenkung für ein Vielpunkt-Meßsystem,
- Fig.8: einen Längsschnitt durch einen Wechselkopf für 90°-Umlenkung im Bereich des Verbundprismas für Beleuchtung und Meßstrahl,
- Fig.9: einen Längsschnitt durch einen Wechselkopf für 90°-Umlenkung im Bereich der Abbildungsmittel,
- Fig.10: eine Ansicht mit Anbaueinheit zur Laserlichtversorgung am Bedienteil,
- Fig.11: eine distalseitige Aufsicht auf ein Einführungsteil mit Arbeitskanal und

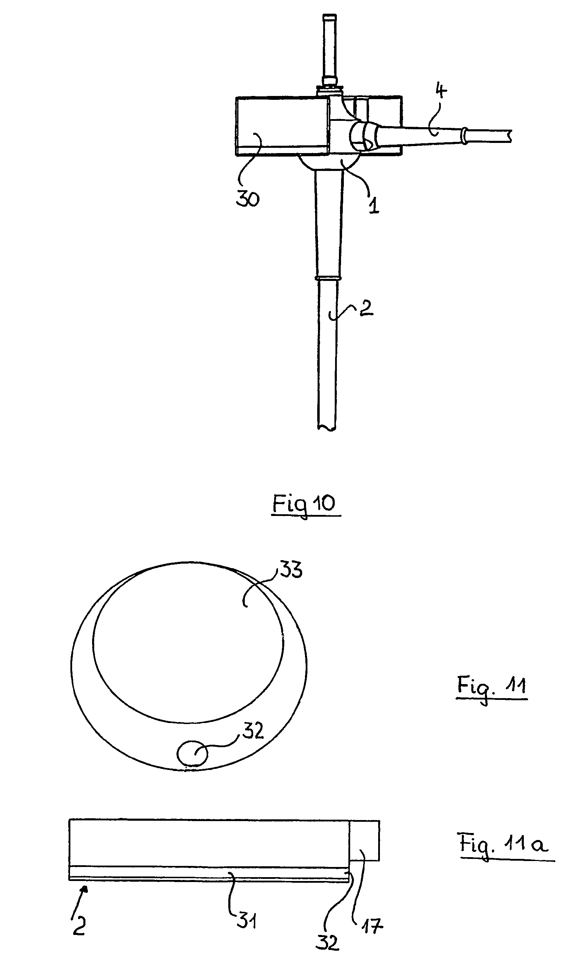

- Fig.11 a: einen Längsschnitt durch ein Einführungsteil mit Arbeitskanal.

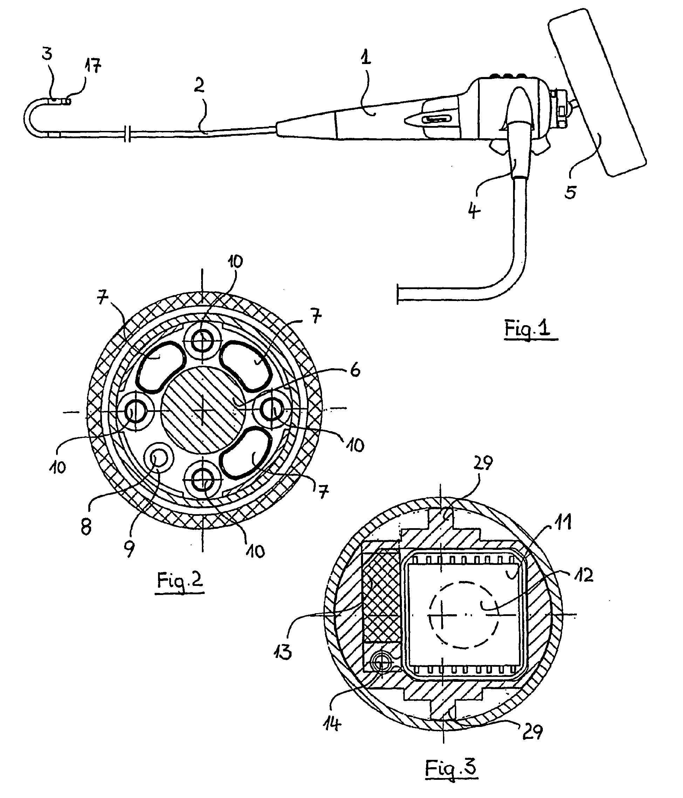

Die in Fig.1 dargestellte Sonde eines endoskopischen Video-Meßsystem besteht aus einem Bedienteil 1, einem Einführungsteil 2, einem distalen Endteil 3 und einem Wechselkopf 17. Das vollständige Meßsystem besteht neben der Sonde noch aus einem nicht dargestellten Multifunktions-Konsolengerät für Lichtquelle, Kamera-Controlling Einheit, Tastatur, Hauptmonitor und Computer für Bildverarbeitung, Meßfunktion und Lasersteuerung, Stromversorgung usw.

An dem Bedienteil 1 ist ein Anschluß 4 zur elektrischen und optischen Versorgung des Meßsystems vorgesehen. Die Bilddarstellung erfolgt über einen Video-Monitor 5. Über weitere an dem Bedienteil 1 vorhandene, nicht näher bezeichnete Knöpfe und Hebel können z.B. die Belichtung geregelt und die Blickrichtung des Kopfteils 3 verändert werden.

Der in Fig.2 dargestellte Querschnitt durch das Einführungsteil 2 zeigt in der Mitte schematisch ein elektrisches Leitungsbündel 6. Darum herum sind z.B. drei Lichtfaserbündel 7 und eine Singlemode-Glasfaser 8 mit Schutzhülle 9 angeordnet. Über vier symmetrisch zueinander angeordnete Bowdenzüge 10 kann die Blickrichtung des distalen Endteils 3 an einem flexiblen distalen Endbereich des Einführungsteils 2 verändert werden.

In dem in Fig.3 dargestellten Querschnitt am distalen Endteil 3 des Einführungsteils 2 sind ein CCD-Bildsensor 11 und eine vorgeschaltete Abbildungsoptik 12 und Bajonett-Zapfen 29 zu sehen. Daneben ist ein Prisma 13 angeordnet, das rückseitig mit den Lichtfaserbündeln 7 gekoppelt ist und über das die Objektbeleuchtung erfolgt. Unterhalb des Prismas 13 ist eine Kollimationsoptik 14 zur Erzeugung eines kollimierten Meßstrahlenbündels 15 angeordnet. Die optischen Abbildungs- und Umlenkungsmittel zur Umformung des Meßstrahlenbündels sind in dem hier nicht weiter dargestellten Wechselkopf 17 enthalten.

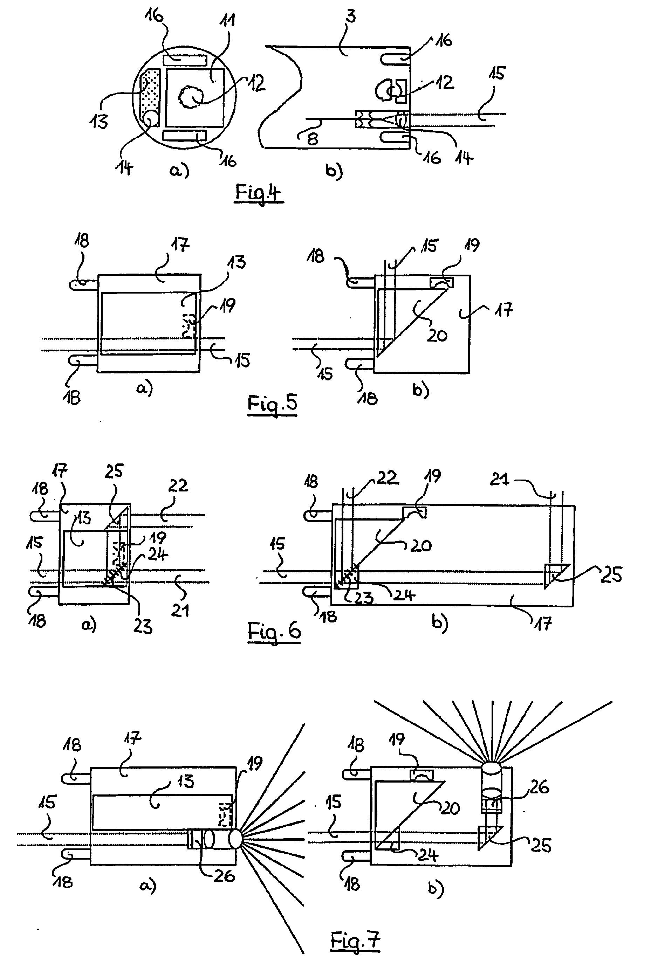

Fig.4a zeigt eine Aufsicht auf das distale Endteil 3 des Einführungsteils 2 nach der Erfindung, wobei die bereits zu Fig.3 beschriebenen Teile gleich bezeichnet sind. Der Singlemode-Glasfaser 8 ist ein optisches System 14 zur Erzeugung eines kollimierten Meßstrahlenbündels 15 zugeordnet, wie aus Fig. 4b ersichtlich ist. Das Kollimationssystem kann dabei eine Gradientenlinse (GRIN) und/oder eine asphärische Linse mit minimaler sphärischer Aberration enthalten. In dem distalen Endteil 3 des Einführungsteils 2 sind Vertiefungen 16 vorgesehen, die zum positionsgenauen Ansetzen von Wechselköpfen mit daran angesetzten Paßstiften korrespondieren.

Mit dem aus dem distalen Endteil 3 kollimiert austretenden Meßstrahlenbündel 15 bildet das endoskopische Video-System bereits ein funktionsfähiges Einstrahl-Meßsystem, allerdings mit minderer Bildqualität. Der Abstand zum Objekt ist durch die Parallaxen-Bewegung des Lichtpunktes im Bild des Objektfeldes eindeutig bestimmbar. Dadurch kann der Abbildungsmaßstab bei einer ebenen, rechtwinklig zur Blickrichtung orientierten Objektfläche definiert werden.

Mit dem ermittelten Abbildungsmaßstab kann eine Längenbestimmung im Bild durchgeführt werden.

Durch Ansetzen eines in Fig.5a dargestellten Wechselkopfes 17 mit Paßstiften 18 kann bei einer 0°-Durchleitung des Meßstrahlenbündels 15 wiederum ein Einstrahl-Meßsystem realisiert werden, jedoch mit deutlich besserer Bildqualität. Die Paßstifte 18 korrespondieren mit den Vertiefungen 16 in der Endfläche des Einführungsteils 2 und sichern die gegenseitige Ausrichtung zwischen distalem Endteil 3 und Wechselkopf 17.

Die Abbildungsoptik zur Wahl des Gesichtsfeldes ist schematisch als distale Eintrittslinse 19 dargestellt. Mögliche nachfolgende Linsen und Prismen sind der Einfachheit halber hier und in den nachfolgenden Ausführungsbeispielen weggelassen. Die Leitung des Beleuchtungslichts soll im quaderförmigen Prisma 13 erfolgen. Im Ausführungsbeispiel nach Fig.5b wird durch das 90°-Prisma 20 das Beleuchtungslicht um 90° abgelenkt. In beiden Ausführungsformen wird das Meßstrahlenbündel 15 ebenfalls durch die Prismen 13, 20 geführt.

Fig.6 zeigt eine Variante des Wechselkopfes 17, bei der aus dem Meßstrahlenbündel 15 zwei parallel zueinander ausgerichtete Meßstrahlenbündel 21, 22 gebildet werden. In der 0°-Durchführung nach Fig.6a enthält das quaderförmige Prisma 13 distalseitig eine unter 45° zur Strahlrichtung stehende Anschrägung 23, auf die ein erstes 90°-Umlenkprisma 24 aufgekittet ist. Die Kittfläche enthält z.B. eine metallische Verspiegelung, die 50 % des Laserlichts unter 90° zur Meßstrahlenrichtung auf ein weiteres 90°-Umlenkprisma 25 richtet, das auf das quaderförmige Prisma 13 ohne Verspiegelung in der Kittschicht aufgekittet ist und das weitere Meßstrahlenbündel 22 an seiner Basisfläche parallel zum ersten Meßstrahlenbündel 21 reflektiert.

Dieses tritt durch die metallisch verspiegelte Anschrägung 23 und das erste Umlenkprisma 24 hindurch.

Bei einer metallischen Verspiegelung ist das Teilungsverhältnis unabhängig von der Polarisationsrichtung des auftreffenden Laserstrahls. Als Singlemode-Glasfaser wird dabei eine nichtpolarisationserhaltende Faser verwendet.

Bei Verwendung einer polarisationserhaltenden Singlemode-Glasfaser kann eine dielektrische, polarisierende Teilerschicht vorgesehen werden. Die Singlemode-Glasfaser ist dabei so im distalen Endteil 3 zu befestigen, daß bei aufgesetztem Wechselkopf 17 die Polarisationsrichtung der Teilerschicht unter 45° zur Polarisationsrichtung des transmittierten Meßstrahlenbündels steht. Die Verwendung senkrecht zueinander polarisierter Meßstrahlenbündel erzeugt im Zusammenwirken mit bestimmten Objektoberflächen besondere Effekte.

In der 90°-Version nach Fig.6b ist ein entsprechendes erstes 90°-Umlenkprisma 24 auf das 90°-Prisma 20 aufgekittet, wobei die Kittfläche wiederum mit 50 % Durchlaß verspiegelt ist, so daß in Transmission das erste Meßstrahlenbündel 21 und in Reflexion das weitere Meßstrahlenbündel 22 entstehen. Das durch das erste Umlenkprisma 24 hindurch tretende erste Meßstrahlenbündel 21 wird über ein weiteres 90°-Umlenkprisma 25 parallel zum weiteren Meßstrahlenbündel 22 ausgesendet. Auf eine entsprechende Ausrichtung des Umlenkprismas 25 muß bei der Montage geachtet werden. Über den Abstand des weiteren Umlenkprismas 25 zum ersten Umlenkprisma 24 kann die Meßbasis des Parallelstrahl-Meßsystems in einfacher Weise beeinflußt werden. Eine vergrößerte Maßbasis erhöht insbesondere bei größeren Arbeitsabständen die Meßgenauigkeit.

Das in Fig.7 dargestellte Ausführungsbeispiel eines Wechselkopfes 17 zeigt die Verwendung eines diffraktiven optischen Elements 26 (DOE) für eine Strahlvervielfachung in mehrere divergierende kollimierte Strahlenbündel. Die Funktionsweise eines solchen Vielpunkt-Meßsystems ist bekannt. Eine Veränderung des Punktemusters ist durch Austauch des DOE 26 möglich. Eine 0°-Durchführung nach Fig.7a oder eine 90°-Umlenkung nach Fig.7b sind mit denselben Mitteln möglich wie in Fig 6 dargestellt und beschrieben.

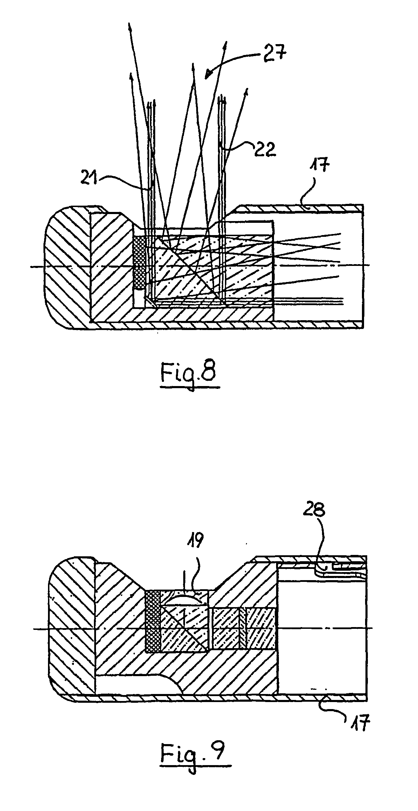

Das in Fig.8 dargestellte Ausführungsbeispiel zeigt einen Längsschnitt durch einen Wechselkopf 17 in der 90°-Umlenkversion im Bereich der Beleuchtungsstrahlen 27 und der Meßstrahlenbündel 21, 22. Die in Fig.6b beschriebenen Umlenk- und Übertragungsmittel sind hier zu einem kompakten Verbundprisma zusammengesetzt.

Der in Fig. 9 dargestellte Längsschnitt an demselben Wechselkopf 17 beinhaltet die distale Eintrittslinse 19 und Umlenkmittel zur Übertragung der Abbildungsstrahlen. Der Wechselkopf 17 ist auf das distale Endteil 3 des Einführungsteiles 2 aufgeschoben und wird z.B. über eine Bajonettverriegelung 28 ausgerichtet und fixiert. Die zugehörigen Bajonett-Zapfen 29 sind hier nicht dargestellt (vgl. Fig.3).

Die in den Fig. 4 bis 7 gezeigten Prinzipdarstellungen machen die Vielzahl von Gestaltungsmöglichkeiten für die Wechselköpfe deutlich, die sich allein daraus ableiten, daß das distalseitig aus dem Einführungsteil austretende Meßstrahlenbündel kollimiert ist. Weitere Projektionsprinzipien, welche die kollimierten Laserstrahlen im Wechselkopf in ein Projektionsmuster verwandeln, können z.B. sein:
a) Antiparallele Strahlen:
   Es können beim Parallelstrahl-Meßsystem die beiden Strahlenbündel antiparallel, bzw. sich schneidend ausgerichtet sein, um z.B. als Index für einen vordefinierten Arbeitsabstand zu dienen.
   Ein vorbestimmter Arbeitsabstand und damit ein bestimmter Abbildungsmaßstab wird erreicht, wenn die beiden Punkte zu einem einzigen Punkt zusammenfallen.
b) Projektion von linearen Targets mit Reflektions-, Transmissions- und Beugungseffekten:
   Wenn das kollimierte, einfallende Strahlenbündel im Wechselkopf mit einer Linse fokussiert wird, dann entsteht im Fokus eine punktförmige Lichtquelle mit physikalisch bedingter Ausdehnung im Bereich von nur 1 bis 3 Wellenlängen. Wird distal des Fokus ein Draht oder eine transparente Faser positioniert, so kann je nach Wahl der Durchmesser und Abstände ein Schattenmuster mit überlagerten Beugungs- und Interferenzerscheinungen erzeugt werden. Das Muster besteht aus einer dunklen Schattenzone, deren beide Begrenzungen aufgrund von Beugungseffekten einen überhellten Lichtsaum aufweisen. Damit entsteht ein wesentlich stärkerer Kontrastveriauf im Bild als bei bekannten Schattenprojektionen. Durch den stärkeren Kontrast und durch die überhellten Schattenränder ist eine genauere Erkennung der Lage des Musters und somit eine genauere Messung möglich. Wird zudem bei der Messung auf das unabhängig schaltbare weiße Beleuchtungslicht verzichtet, so kann der Kontrast weiter gesteigert werden.
c) Projektion eines flächenhaften Targets:
   Der kollimierte Laserstrahl wird im Wechselkopf fokussiert. Der Fokus beleuchtet wiederum in einem gewissen Abstand z.B. eine flächenhafte Lochmaske oder ein Diapositiv mit beliebigem Muster, dargestellt z.B. durch eine Glasplatte mit metallisierten Abdeckungen. Es können dabei durch geschickte Dimensionierung der Strukturgrößen und des Abstandes zwischen Fokus und Target Beugungseffekte erzwungen werden, welche die Helligkeitskanten im Bild verstärken oder sogar überhöhen.
d) Erzeugung eines Streifenmusters durch Interferenz:
   Das kollimierte Laserstrahlenbündel wird im Wechselkopf mit einer Zylinderlinse fokussiert und durch eine optische Komponente, z.B. ein Wollaston-Prisma, aufgeteilt und in geringem seitlichen Abstand parallel wieder ausgesendet. Bei geringem Abstand der beiden Lichtaustritte aus gemeinsamer Quelle entstehen Interferenzstreifen, die das Projektionsmuster ergeben. Bei Bedarf können die Streifenabstände z.B. durch Veränderung des seitlichen Abstandes der Lichtaustritte variiert werden.
e) Erzeugung beliebiger Muster mittels DOEs:
   Anstelle des Punktmusters kann ein DOE quasi ein beliebiges Muster erzeugen. Beschränkungen in der Wahl des Musters treten erst dann auf, wenn das DOE weitwinklig abstrahlen soll. Denkbar sind einfache Muster wie einzelne Linien und parallele Linienscharen, gekreuzte Linien und gekreuzte parallele Linienscharen (Gitter), Ringe etc. Auch kompliziertere, flächenhafte Muster wie Zufallsmuster oder Muster mit flächenhaften Codierungen sind erzeugbar. Möglich ist auch die Verwendung von zwei oder mehr DOE-Projektoren, die an verschiedenen Positionen um das Kameraobjektiv angelegt sind. So können vertikale und horizontale Streifenscharen von getrennten Projektoren ausgehen.
f) Erzeugung von Speckles:
   Im Wechselkopf kann durch eine einfache Fokussierung eine divergente Beleuchtung erzeugt werden, welche bei der Bildaufnahme zu Speckles führt. Diese Speckles können Teil einer an sich bekannten Meßmethode sein (Electronic Speckle Interferometry).

Die aus Bedienteil 1 und Einführungsteil 2 bestehende Sonde eines Video-Endoskops kann auch auf ein reines Beobachtungssystem reduziert werden, wenn die Singlemode-Glasfaser 8 weggelassen wird. An ihrer Stelle können z.B. ein separates Leerrohr, bzw. die leere Schutzhülle 9 und eine Einsatzöffnung im distalen Endteil 3 des Einführungsteils 2 vorgesehen sein. Über die Einsatzöffung kann eine Singlemode-Glasfaser 8 mit Kollimationsoptik 14 auch nachträglich in das System eingesetzt werden. Das Leerrohr kann außerdem als Arbeitskanal für den Einsatz von Haken, Zangen u.ä. genutzt werden, deren Operation im Objektbereich durch das Videosystem beobachtet werden kann. Die Modularität des Systems wird auf diese Weise nochmals erweitert.

Dabei ist es vorteilhaft, wenn für die Einspeisung des Laserlichts in die Singlemode-Glasfaser 8 am Bedienteil 1 oder einer damit verbundenen Steckereinheit eine separate Eintrittsöffnung vorgesehen ist, da der Anschluß 4 zur übrigen optischen und elektrischen Versorgung dann bei einer Nachrüstung zu einem Meßsystem davon unberührt bleiben kann. Die Laserlichtquelle kann dazu in einem separaten Gehäuse angeordnet sein, das in Form einer das Bedienteil 1 oder eine separate Steckereinheit umgreifenden Anbaueinheit 30 ausgebildet sein kann. Eine solche Anbaueinheit 30 ist in Fig.10 an dem Bedienteil 1 schematisch dargestellt.

Als Laserlichtquelle kann insbesondere eine im sichtbaren Wellenlängenbereich strahlende Laserdiode vorgesehen sein. Vorzugsweise sollte die Laserdiode bei 639 nm ±3 nm arbeiten. Zur Ankopplung der Singlemode-Glasfaser 8 an die Laserdiode kann in der Anbaueinheit 30 eine lösbare Steckverbindung vorgesehen sein. Durch die Lösbarkeit der Verbindung werden Reparaturen erleichtert.

Die verwendete Laserdiode und/oder die Laserdioden-Ansteuerung sollten so ausgelegt sein, daß Speckles im Strahlungsfeld reduziert sind. Dazu können Speckles reduzierende Mittel zwischen Laserdiode und Eintrittsfläche der Singlemode-Glasfaser 8 angeordnet werden. Die Erkennbarkeit des Meßmusters auf dem Objekt wird durch Unterdrückung von Speckles deutlich verbessert.

Bei den in den Ausführungsbeispielen bisher dargestellten Einführungsteilen 2, distalen Endteilen 3 und Wechselköpfen 17 weisen alle Teile im wesentlichen dieselben Dimensionierungen im Querschnitt auf. Die Kopplungsfläche des Wechselkopfes 17 liegt symmetrisch zur Fläche des distalen Endteiles 3.

Wie in den Fig.11, 11a dargestellt, ist es jedoch auch möglich, im peripheren Randbereich des Einführungsteiles 2 einen zusätzlichen Arbeitskanal 31 zum Einführen von besonderen mechanischen Manipulationsmitteln, wie Haken und Zangen, vorzusehen. Der Querschnitt des Einführungsteiles 2 im Bereich des distalen Endteiles 3 ist dann so groß zu wählen, daß die distalseitige Öffnung 32 des Arbeitskanals 31 unverdeckt neben dem Wechselkopf 17 liegt. Die Zentrier- und Kopplungsmittel zum Ansetzen des Wechselkopfes 17 im Kopplungsbereich 33 sind entsprechend asymmetrisch am distalen Endteil 3 anzuordnen. Die im Arbeitskanal 31 geführten Instrumente können auf diese Weise unbehindert am Wechselkopf 17 vorbei zu dem beobachteten Objektbereich geführt werden.

### Bezugszeichenliste

- 1: Bedienteil
- 2: Einführungsteil
- 3: distales Endteil
- 4: Anschluß zur optischen und elektrischen Versorgung
- 5: Video-Monitor
- 6: Leitungsbündel
- 7: Lichtfaserbündel
- 8: Singlemode-Glasfaser
- 9: Schutzhülle
- 10: Bowdenzüge
- 11: CCD-Bildsensor
- 12: Abbildungsoptik
- 13: Prisma
- 14: optisches System zur Kollimation
- 15: kollimiertes Meßstrahlenbündel
- 16: Vertiefungen
- 17: Wechselkopf
- 18: Paßstifte
- 19: distale Eintrittslinse
- 20: 90°-Prisma
- 21: erstes Parallelstrahl-Bündel
- 22: zweites Parallelstrahl-Bündel
- 23: Anschrägung mit Teilverspiegelung
- 24: erstes 90°-Umlenkprisma
- 25: weiteres 90°-Umlenkprisma
- 26: diffraktives optisches Element (DOE)
- 27: Beleuchtungsstrahlen
- 28: Bajonettverriegelung
- 29: Bajonett-Zapfen
- 30: Anbaueinheit
- 31: Arbeitskanal
- 32: distalseitige Öffnung
- 33: Kopplungsbereich

## Patentansprüche

1. Endoskopisches Video-Meßsystem mit einem proximalen Bedienteil (1), einem Einführungsteil (2) und einem daran ansetzbaren Wechselkopf (17), wobei das Bedienteil (1) einen Anschluß (4) zur elektrischen und optischen Versorgung des Systems enthält, im Einführungsteil (2) optische Übertragungsmittel für die Objektbeleuchtung (7) und elektrische Versorgungs- und Übertragungsleitungen (6) für einen im distalen Endteil (3) angeordneten elektronischen Bildsensor (11) vorgesehen sind und der Wechselkopf (17) optische Übertragungsmittel für die Objektbeleuchtung (13) und die Objektabbildung (12) enthält, **dadurch gekennzeichnet, daß** im Einführungsteil (2) zur Übertragung einer Meßstrahlung eine Singlemode-Glasfaser (8) vorgesehen ist, der ein im distalen Endteil (3) des Einführungsteiles (2) angeordnetes optisches System (14) zur Erzeugung eines kollimierten Meßstrahlenbündels (15) zugeordnet ist.

2. Endoskopisches Video-Meßsystem nach Anspruch 1, **dadurch gekennzeichnet, daß** dem kollimierten Meßstrahlenbündel (15) ein Wechselkopf (17) mit einem optischen Teilerelement (23) und mindestens einem 90°-Umlenkprisma (24, 25) zur Erzeugung eines ersten (21) und eines zweiten (22) parallel zueinander aus dem Wechselkopf (17) austretenden kollimierten Meßstrahlenbündels zugeordnet ist.

3. Endoskopisches Video-Meßsystem nach Anspruch 1, **dadurch gekennzeichnet, daß** dem kollimierten Meßstrahlenbündel (15) ein Wechselkopf (17) mit einem diffraktivem optischem Element (DOE) (26) zur Erzeugung eines aus dem Wechselkopf (17) austretenden, ein Meßpunktemuster bildenden Vielstrahlenbündels zugeordnet ist.

4. Endoskopisches Video-Meßsystem nach Anspruch 1, **dadurch gekennzeichnet, daß** zur Versorgung der Singlemode-Glasfaser (8) eine in einem separaten Gehäuse angeordnete Laserlichtquelle vorgesehen ist, wobei das Gehäuse in Form einer das Bedienteil (1) umgreifenden Anbaueinheit (30) ausgebildet ist.

5. Endoskopisches Video-Meßsystem nach Anspruch 1, **dadurch gekennzeichnet, daß** im peripheren Randbereich des Einführungsteiles (2) ein zur Durchführung mechanischer Manipulationsmittel geeigneter Arbeitskanal (31) vorgesehen ist, dessen distalseitige Öffnung (32) außerhalb des Kopplungsbereichs (33) des Wechselkopfes (17) am distalen Endteil (3) liegt.

## Claims

1. Endoscopic video measurement system with a proximal operating portion (1), an insertion portion (2) and an interchangeable head (17) which can be attached thereto, wherein the operating portion (1) comprises a connection (4) for the electrical and optical supply for the system, optical transmission means for the object illumination (7) and electrical supply and transmission lines (6) for an electronic image sensor (11), which is arranged in the distal end portion (3), are provided in the insertion portion (2), and the interchangeable head (17) comprises optical transmission means for the object illumination (13) and the object imaging (12), **characterized in that** a single-mode glass fibre (8), which has associated with it an optical system (14) arranged in the distal end portion (3) of the insertion portion (2) for producing a collimated measurement beam bundle (15), is provided in the insertion portion (2) for transmitting a measurement radiation.

2. Endoscopic video measurement system according to Claim 1, **characterized in that** the collimated measurement beam bundle (15) has associated with it an interchangeable head (17) with an optical splitter element (23) and at least one 90° deflection prism (24, 25) for producing a first (21) and a second (22) collimated measurement beam bundle, which beam bundles exit from the interchangeable head (17) in a mutually parallel manner.

3. Endoscopic video measurement system according to Claim 1, **characterized in that** the collimated measurement beam bundle (15) has associated with it an interchangeable head (17) with a diffractive optical element (DOE) (26) for producing a multi-beam bundle which exits from the interchangeable head (17) and forms a measurement point pattern.

4. Endoscopic video measurement system according to Claim 1, **characterized in that** a laser light source which is arranged in a separate housing is provided for the supply to the single-mode glass fibre (8), wherein the housing is in the form of an add-on unit (30) encompassing the operating portion (1).

5. Endoscopic video measurement system according to Claim 1, **characterized in that** a working channel (31), which is suitable for guiding mechanical manipulation means through it and whose distal-end opening (32) lies outside the coupling region (33) of the interchangeable head (17) at the distal end portion (3), is provided in the peripheral edge region of the insertion portion (2).

## Revendications

1. Système de mesure vidéo endoscopique comprenant une partie de commande (1) proximale, une partie à introduire (2) et une tête interchangeable (17) pouvant être montée sur celle-ci, la partie de commande (1) comprenant un raccord (4) pour l'alimentation électrique et optique du système, des moyens de transmission optiques étant prévus dans la partie à introduire (2) pour l'éclairage de l'objet (7) ainsi que des lignes d'alimentation et de transmission (6) électriques pour un capteur d'image (11) électronique disposé dans la partie d'extrémité (3) distale et la tête interchangeable (17) contenant des moyens de transmission optiques pour l'éclairage de l'objet (13) et la représentation de l'objet (12), **caractérisé en ce qu'**une fibre optique monomodale (8) est prévue dans la partie à introduire (2) pour transmettre un rayonnement de mesure, à laquelle est associé un système optique (14) destiné à générer un faisceau de rayons de mesure (15) collimaté et disposé dans la partie d'extrémité (3) distale de la partie à introduire (2).

2. Système de mesure vidéo endoscopique selon la revendication 1, **caractérisé en ce qu'**au faisceau de rayons de mesure (15) collimaté est affectée une tête interchangeable (17) munie d'un élément séparateur optique (23) et d'au moins un prisme de déviation de 90° (24, 25) pour produire un premier (21) et un deuxième (22) faisceaux de rayons de mesure collimatés qui sortent parallèlement l'un à l'autre de la tête interchangeable (17).

3. Système de mesure vidéo endoscopique selon la revendication 1, **caractérisé en ce qu'**au faisceau de rayons de mesure (15) collimaté est affectée une tête interchangeable (17) munie d'un élément optique diffractif (DOE) (26) pour produire un faisceau de rayons multiples sortant de la tête interchangeable (17) et formant un modèle de point de mesure.

4. Système de mesure vidéo endoscopique selon la revendication 1, **caractérisé en ce que** pour alimenter la fibre optique monomodale (8), il est prévu une source de lumière laser disposée dans un boîtier séparé, le boîtier étant réalisé sous la forme d'une pièce rapportée (30) entourant la partie de commande (1).

5. Système de mesure vidéo endoscopique selon la revendication 1, **caractérisé en ce que** dans la zone de bordure périphérique de la partie à introduire (2) est prévu un canal de travail (31) conçu pour le passage de moyens de manipulation mécaniques dont l'ouverture (32) côté distal se trouve sur la partie d'extrémité (3) distale à l'extérieur de la zone d'accouplement (33) de la tête interchangeable (17).
